# EUROPEAN PATENT APPLICATION

(11) **EP 4 129 202 A1**
(43) Date of publication of application: **08.02.2023**
(21) Application number: 21775484.5
(22) Date of filing: 11.01.2021
(51) Int. Cl.: A61B 17/00, A61B 17/34, A61F 2/82

(54) **ANCHOR AND ANCHORING DEVICE**

(30) Priority: 27.03.2020 CN 202010230037
(71) Applicant: MicroPort Urocare (Jiaxing) Co., Ltd., Nanhu District Jiaxing Zhejiang 314006 (CN)
(72) Inventor: WANG, Zhen, Jiaxing, Zhejiang 314006 (CN); CUI, Yujia, Jiaxing, Zhejiang 314006 (CN); FU, Jiawei, Jiaxing, Zhejiang 314006 (CN); HU, Junfeng, Jiaxing, Zhejiang 314006 (CN)
(74) Representative: Marks & Clerk LLP
(86) International application number: PCT/CN2021/071067
(87) International publication number: WO 2021/190092

(57) **Abstract**

An anchor (100) and an anchoring device. The anchoring device comprises the anchor (100), and the anchor (100) comprises a first anchoring part (110) and a second anchoring part (120) extending out from one end of the first anchoring part (110); the second anchor part (120) comprises a first anchoring sheet (121) and a second anchoring sheet (122) which are spaced apart from each other, and a clamping groove is formed between the first anchoring sheet (121) and the second anchoring sheet (122); in a direction from the first anchoring part (110) to the second anchoring part (120), the clamping groove comprises a buffer section (131), a clamping section (132), and a first transition section (133); the width of the clamping section (132) is greater than that of the buffer section (131), the width of the first transition section (133) gradually increases in a direction away from the clamping section (132), and the minimum width of the first transition section (133) is smaller than the width of the clamping section (132). The anchoring device has a good anchoring effect, and has a low risk of displacement in prostatic tissue when treating benign prostatic hyperplasia.

## Description

### TECHNICAL FIELD

The present invention relates to the technical field of medical instruments and, in particular, to an anchor and an anchoring device.

### BACKGROUND

Benign prostatic hyperplasia (BPH) is the most common benign disease that causes urination disorders in middle-aged and elderly men. The main manifestations are histological hyperplasia of prostatic stromal and glandular components, anatomical enlargement of the prostate, clinical symptoms dominated by lower urinary tract symptoms (LUTS), and urodynamic bladder outlet obstruction (BOO).

In addition to drug treatment, the surgical methods for benign prostatic hyperplasia include: (1) transurethral resection of the prostate; (2) suprapubic or retropubic prostatectomy; (3) laser enucleation or resection of the prostate; (4) minimally invasive treatment using prostate stents.

An anchoring device, which is a permanent implant used to relieve a small flow of urine, is commonly used in the minimally invasive treatment using prostate stents. This anchoring device configured to be implanted into the urethra by a delivery system, and after implantation, the left and right lobes of the prostate are mechanically separated to push away the prostate tissue that compresses the urethra, thereby achieving a therapeutic effect. This kind of anchoring device is extremely low in invasiveness and does not require ablation, can continuously relieve the symptoms of patients, and has a good therapeutic effect.

Currently, anchoring devices generally include a distal anchor, a proximal anchor, and a connector, and the connector is connected to the distal anchor and the proximal anchor, respectively. This anchoring device has a risk of displacement after implantation, and the effectiveness and stability of the treatment need to be improved.

### SUMMARY OF THE INVENTION

An object of the present invention is to provide an anchor and an anchoring device to solve the problem that the existing anchor and anchoring device are likely to displace after implantation.

To achieve the above object, the present invention provides an anchor, comprising a first anchoring part and a second anchoring part extending from an end of the first anchoring part;
wherein the second anchoring part comprises a first anchoring sheet and a second anchoring sheet that are spaced apart from each other, and a clamping groove is formed between the first anchoring sheet and the second anchoring sheet; wherein the clamping groove comprises a buffer section, a clamping section and a first transition section that are arranged in sequence along a direction from the first anchoring part to the second anchoring part, the clamping section having a width larger than a width of the buffer section, the first transition section having a width gradually increasing along a direction away from the clamping section, a minimum width of the first transition section is smaller than the width of the clamping section; wherein the width refers to a distance between the anchoring sheet and the second anchoring sheet.

Optionally, the clamping groove is configured to clamp a target object, and the buffer section has a length greater than a diameter of a radial section of the target object.

Optionally, the minimum width of the first transition section is smaller than the diameter of the radial section of the target object, and a maximum width of the first transition section is larger than the diameter of the radial section of the target object.

Optionally, in an extension direction of the anchor, a ratio of a length of the first anchoring part to a length of the anchor is in a range of [1/10, 1/2].

Optionally, the clamping groove further comprises an open section that is arranged at an end of the first transition section away from the clamping section and has a width that is equal to a maximum width of the first transition section.

To achieve the above object, the present invention also provides an anchoring device, comprising a connector, a distal anchor and the above anchor, the connector comprising a first connection part and a second connection part, the distal anchor fixedly connected with the first connection part, and the second anchoring part of the anchor configured to clamp the second connection part so that the anchor is connected to the second connection part.

Optionally, the distal anchor comprises an anchoring body and a bent structure extending from an end of the anchoring body, the anchoring body comprising a third anchoring part that is tubular;
wherein the first connection part is arranged in an inner cavity of the third anchoring part, and the first connection part and the third anchoring part are fixed to each other by crimping, so that a wall of the third anchoring part extends into the inner cavity to form a limiting recess, and a fixing groove matching with the limiting recess is formed on the first connection part.

Optionally, a limiting part is provided at an end of the first connection part away from the second connection part, the limiting portion has a diameter larger than a diameter of the inner cavity, and the limiting part is located outside the inner cavity of the third anchoring part, so as to prevent the first connection part from moving out of the inner cavity of the third anchoring part.

Optionally, the anchoring body further comprises a fourth anchoring part having a curved sheet-like structure and configured to connect the third anchoring part and the bent structure; at a connection between the fourth anchoring part the third anchoring part, the fourth anchoring part covers at least 1/2 of a circumferential surface of the anchoring body.

Optionally, a portion of the circumferential surface that the fourth anchoring part covers gradually reduces in a direction away from the third anchoring part.

Optionally, the bent structure comprises a bent part; at the connection between the bent part and the fourth anchoring part, the fourth anchoring part covers at least 1/12 of the circumferential surface of the anchoring body.

Optionally, the bent structure further comprises a fifth anchoring part arranged at an end of the bent part away from the fourth anchoring part; an angle in a range of [90°, 180°) is formed between the fifth anchoring part and an axis of the anchoring body.

Optionally, the bent structure is formed as a curved sheet-like structure, and the fifth anchoring part covers at least 1/2 of a circumferential surface of the bent structure.

Optionally, in an axial direction of the bent structure, the fifth anchoring part has a length of 0.5mm-1mm.

Optionally, in an axial direction of the anchoring body, a ratio of a length of the third anchoring part to a length of the anchoring body is in a range of [1/10, 1/2].

Compared with the prior art, the anchor and anchoring device of the present invention have the following advantages.

The aforementioned anchor includes a first anchoring part and a second anchoring part extending from an end of the first anchoring part. The second anchoring part includes a first anchoring sheet and a second anchoring sheet that are spaced apart from each other. A clamping groove is formed between the first anchoring sheet and the second anchoring sheet. Along the direction from the first anchoring part to the second anchoring part, the clamping groove includes a buffer section, a clamping section and a first transition section that are arranged in sequence. The width of the clamping section is greater than the width of the buffer section, and the width of the first transition section gradually increases along the direction away from the clamping section. The minimum width of the first transition segment is smaller than the width of the clamping section. The width refers to the distance between the first anchoring sheet and the second anchoring sheet. when the anchor is applied to the anchoring device, it is used as a proximal anchor. The anchoring device further includes a connector. The second anchoring part is configured to clamp the connector, so that the connector is connected with the anchor. The buffer section is used to avoid the bifurcation and formation of the first transition section during the process of snapping the connector into the clamping section. After the anchoring device is implanted in the patient's body, the connector can be at least partially clamped by the buffer section, so that the connector can move to the clamping section when a part of the anchoring device is displaced, to ensure that the connector is still connected with the anchor, thereby improving the reliability of the anchoring device.

### BRIEF DESCRIPTION OF THE DRAWINGS

Fig. 1 is a schematic structural diagram of an anchoring device according to an embodiment of the present invention;
Fig. 2 is a schematic structural diagram of the proximal anchor of the anchoring device viewed from a direction according to an embodiment of the present invention;
Fig. 3 is a schematic structural diagram of the proximal anchor of the anchoring device in Fig. 2 viewed from another direction;
Fig. 4 is a schematic diagram of the anchoring device according to an embodiment of the present invention, in which the connector is snapped into the second anchoring part of the proximal anchor;
Fig. 5 is a schematic diagram of the anchoring device according to an embodiment of the present invention, in which the connector is snapped into the clamping section;
Fig. 6 is a schematic diagram of the anchoring device according to an embodiment of the present invention, in which the connector is snapped into the buffer section;
Fig. 7 is a schematic cross-sectional view perpendicular to the X direction, showing the proximal anchor in the anchoring device according to an embodiment of the present invention;
Fig. 8 is a schematic structural diagram of a distal anchor in the anchoring device according to an embodiment of the present invention;
Fig. 9 is a schematic diagram showing a connection between the connector and the distal anchor of the anchoring device according to an embodiment of the present invention;
Fig. 10 is a schematic diagram of implanting in a patient a plurality of anchoring devices according to embodiments of the present invention;
Figs. 11a to 11g are schematic diagrams of the process of implanting into tissue the anchoring device according to an embodiment of the present invention;
Fig. 12 is a schematic view of the distal end of the connector of the anchoring device according to an embodiment of the present invention, in which the distal end is formed as a tip;
Fig. 13 is a schematic structural diagram of an auxiliary tool for assembling the connector and the distal anchor according to an embodiment of the present invention, in which the heating wire is not shown;
Fig. 14 is a cross-sectional view of a processing part of the auxiliary tool for assembling the connector and the distal anchor according to an embodiment of the present invention;
Fig. 15 is a schematic diagram of the auxiliary tool for assembling the connector and the distal anchor according to an embodiment of the present invention, in which crimping and hot-melting processes are performed at the same time;
Fig. 16 is a schematic diagram showing the forces that the anchoring device is subject to when the connector and the distal anchor are assembled by using the auxiliary tool according to an embodiment of the present invention.

List of reference signs:
100: proximal anchor;
110: first anchoring part;
120: second anchoring part;
121: first anchoring sheet, 122: second anchoring sheet;
131: buffer section, 132: clamping section, 133: first transition section, 134: open section;
200: connector;
210: first connection part;
220: second connection part;
221: tip;
230: limiting part;
300: distal anchor;
310: third anchoring part;
311: limiting recess;
320: fourth anchoring part;
321: indent;
330: bent part;
340: fifth anchoring part;
10: sheath, 20: distal anchor delivery device, 30: paracentesis needle, 40: proximal anchor delivery device;
1000: auxiliary tool;
1100: fixture;
1110: sub-fixture;
1111: base, 1112: clamping arm;
1200: processing part;
1210: crimping section; 1220: heating section;
2000: heating wire.

### DETAILED DESCRIPTION

To make the objectives, advantages and characteristics of the present invention clear, the present invention will be further described in detail in conjunction with the drawings. It should be noted that the drawings are in very simplified form and not made to scale, and only intent to facilitate a clear illustration for embodiments of the invention.

As used in this specification, the singular forms "a", "an", and "the" include plural referents, and the plural form "a plurality" includes two or more referents unless the specification clearly dictates otherwise. As used in this specification, the term "or" is generally employed in its sense including "and/or" unless the specification clearly dictates otherwise, and the terms "installed", "coupled", "connected" shall be understood in a broad sense, for example, it may be a fixed connection, a detachable connection, or an integral connection. It can be a mechanical connection or an electrical connection. It can be directly connected, or indirectly connected through an intermediate medium, and it can be the internal communication between two elements or the interaction between the two elements. For those of ordinary skill in the art, the specific meanings of the above terms in the present invention can be understood according to specific situations.

As used herein, a "proximal end" and a "distal end" are relative orientations, relative positions and directions of elements or actions relative to each other from the perspective of the doctor using the medical instruments. Although the "proximal end" and the "distal end" are not restrictive, the "proximal end" generally refers to the end of the medical device that is close to the doctor during normal operation, while the "distal end" generally refers to the end that first enters the patient's body.

Embodiments of the present invention provide an anchor and an anchoring device that can be implanted into prostate tissue to treat benign prostatic hyperplasia, or into other anatomical structures to treat other diseases. For ease of understanding, the embodiments take the use of the anchoring device to treat benign prostatic hyperplasia as an example for introduction. In addition, when the anchor is applied to the anchoring device, the anchor is defined as a proximal anchor, so hereinafter, the "proximal anchor" will always be used to refer to the anchor.

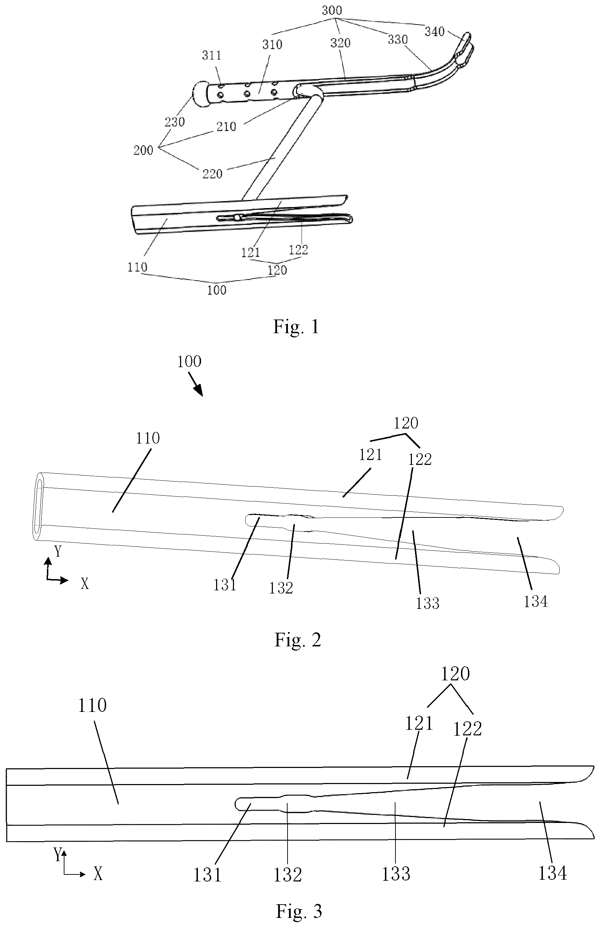

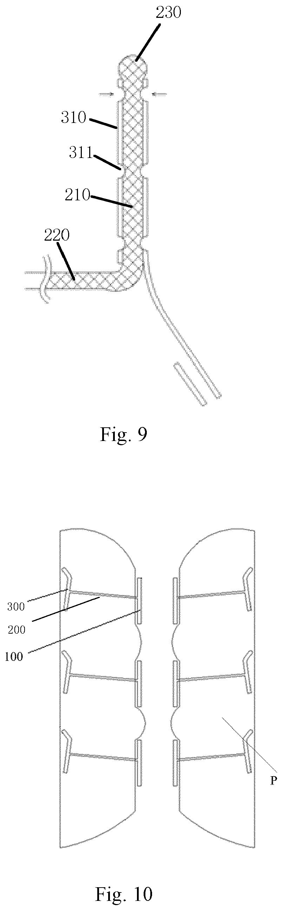

Please refer to Fig. 1 in conjunction with Fig. 9, the anchoring device includes a proximal anchor 100, a connector 200 and a distal anchor 300. The connector 200 includes a first connection part 210 and a second connection part 220. The first connection part 210 is fixedly connected with the distal anchor 300, and the second connection part 220 is selectively connected with the proximal anchor 100.

Referring to Figs. 2 and 3, the proximal anchor 100 includes a first anchoring part 110 and a second anchoring part 120 extending from one end of the first anchoring part 110. The second anchoring part 120 includes a first anchoring sheet 121 and a second anchoring sheet 122 that are spaced apart from each other, and a clamping groove is formed between the first anchoring sheet 121 and the second anchoring sheet 122. Along the direction from the first anchoring part 110 to the second anchoring part 120, the clamping groove includes a buffer section 131, a clamping section 132 and a first transition section 133. The width of the clamping section 132 is larger than the width of the buffer section 131, the width of the first transition section 133 gradually increases along the direction away from the clamping section 132, and the minimum width of the first transition section 133 is smaller than the width of the clamping section 132. Here, the "width" refers to the distance between the first anchoring sheet 121 and the second anchoring sheet 122. Taking the direction shown in the figures as an example, the proximal anchor 100 extends along the X direction, and the Y direction may be the width direction of the proximal anchor 100.

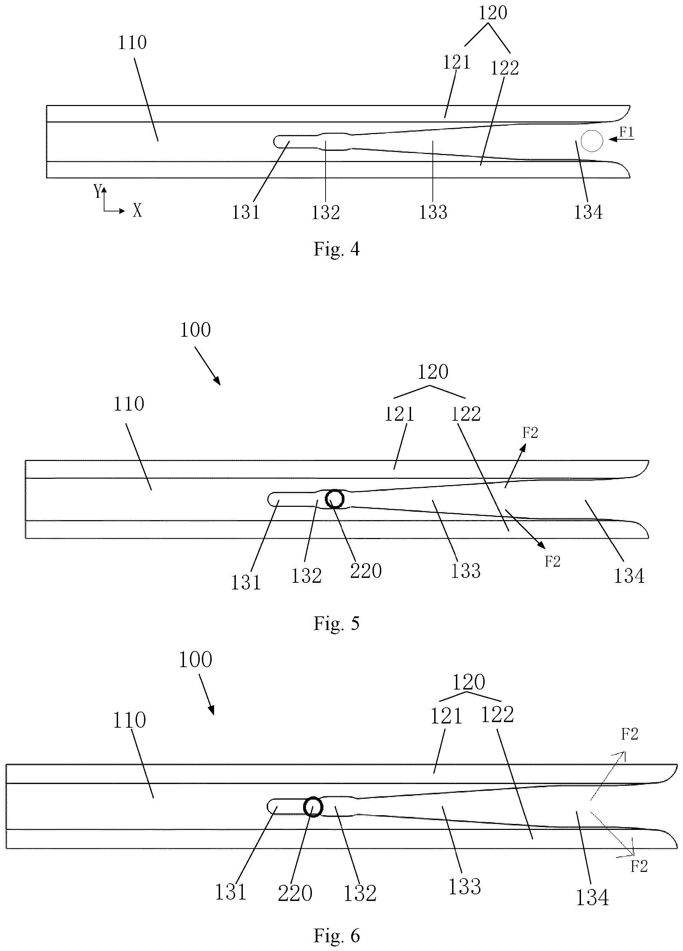

In this embodiment, the clamping groove of the second anchoring part 120 is configured to clamp the proximal end of the second connection part 220 (that is, an end of the second connection part 220 that is away from the first connection part 210, which is also the proximal end of the connector 200. Correspondingly, the end of the first connection part 210 that is away from the second connection part 220 is hereinafter referred to as the distal end of the second connection part 220 or the distal end of the connector 200), so as to achieve the connetion between the proximal anchor 100 and the connector 200. Referring to Fig. 4, when the connector 200 and the proximal anchor 100 are being assembled, the axial direction of the second connection part 220 is perpendicular to the X direction and the Y direction. A force F1 is applied to the second connection part 220, so that the second connection part 220 can enter the clamping groove from the first transition section 133 and be clamped by the second anchoring part at the clamping section 132. As a result, the proximal anchor 100 and the connector 200 are connected, as shown in Fig. 5. A buffer section 131 is defined in the clamping groove. When the proximal anchor 100 and the connector 200 are being assembled, the second connection part 220 enters the clamping section 132 (as shown in Fig. 5), even into the buffer section 131 (shown in Fig. 6), duing which the buffer section 131 provides a buffering effect to prevent the first anchoring sheet 121 and the second anchoring sheet 122 from deformation due to a large force F2 that they are subject to. The width of the clamping section 132 is greater than the width of the buffer section 131. Therefore, when the second connection part 220 is displaced by force to at least partially enter the buffer section 131 (as shown in Fig. 6), the second connection part 220 can still return to the clamping section 132. The minimum width of the first transition section 133 is smaller than the width of the clamping section 132. As a result, a protruding limit structure is formed at the intersection of the clamping section 132 and the first transition section 133. In this way, it is difficult for the second connection part 220 clamped by the clamping section 132 to slide to the first transition section 133, so that the proximal anchor 100 is prevented from being separated from the connector 200.

Preferably, the radial section of the proximal end of the second connection part 220 is generally circular, the length of the buffer section 131 is greater than the diameter of the radial section of the proximal end of the second connection part 220, the minimum width of the first transition section 133 is smaller than the diameter of the radial section of the proximal end of the second connection part 220, and the maximum width of the first transition section 133 is larger than the diameter of the radial section of the proximal end of the second connection part 220. It can be understood that, for the proximal anchor 100, the "length" refers to the dimension of each part of the proximal anchor 100 in the X direction.

Further, please continue to refer to Figs. 2 and 3, the clamping groove further includes an open section 134. The open section 134 is disposed at an end of the first transition section 133 away from the clamping section 132, and has a width that is equal to the maximum width of the first transition section 133. Generally, the first anchoring sheet 121 and the second anchoring sheet 122 have a certain thickness at the open section 134, so as to prevent the prostate tissue from being damaged when the anchoring device is implanted into the prostate of the patient. The "thickness" refers to the dimension of the first anchoring sheet 121 or the second anchoring sheet 122 in the Y direction.

In addition, the first anchoring part 110 should also have a predetermined length to ensure the strength of the proximal anchor 100. The length of the first anchoring part 110 accounts for 1/3-1/2 of the length of the proximal anchor 100.

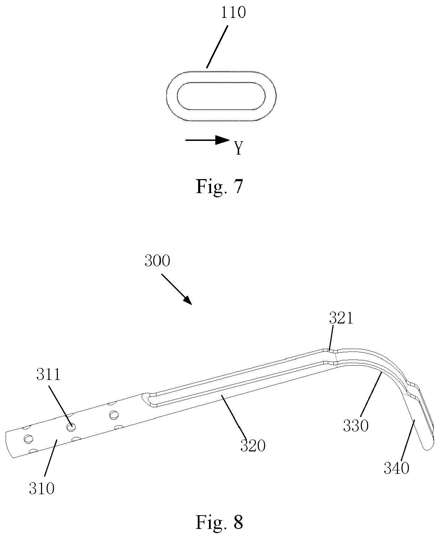

In addition, the cross-section of the proximal anchor 100 perpendicular to the X-direction may be circular, oval, drop-shaped, waist-shaped, etc., but preferably the cross-section is waist-shaped with its length extending along the Y-direction (as shown in Figs. 2 and 7). This has the advantage that when the anchoring device is implanted into the prostate tissue of the patient, the proximal anchor 100 has greater contact area with the prostate tissue, which can improve the anchoring performance.

Please continue to refer to Fig. 1 in conjunction with Fig. 8, the distal anchor 300 includes an anchoring body and a bent structure extending from one end of the anchoring body. The anchoring body includes a third anchoring part 310 that is tubular and is used for connecting with the connector 200.

Specifically, the first connection part 210 is arranged in the inner cavity of the third anchoring part 310, and the first connection part 210 is fixed to the third anchoring part 310 by crimping, so that the wall of the third anchoring part 310 extends into the inner cavity to form limiting recesses 311, and fixing grooves (not annotated in the figures) matching the limiting recesses 311 are formed on the first connection part 210. In the inner cavity of the third anchoring part 310, the limiting recesses 311 protrude from the inner wall of the inner cavity and are matched with the fixing grooves, thereby preventing the connector 200 from moving axially along the inner cavity of the third anchoring part 310 and from rotating relative to the third anchoring part 310, and improving the stability of the connection between the distal anchor 300 and the connector 200, and thus the reliability of the anchoring device in use. On the outer wall of the third anchoring part 310, the limiting recesses 311 are concave inward so as to be able to load drugs to improve the treatment effect of benign prostatic hyperplasia. Preferably, in the axial direction of the anchoring body, the length of the third anchoring part 310 accounts for 1/10-1/2 of the length of the anchoring body.

Referring to Fig. 9, the cross-section of each limiting recess 311 in the direction along which the limiting recess extends in the inner cavity of the third anchoring part 310 is a semicircle. In other embodiments, the cross-section of each limiting recess 311 in the direction along which the limiting recess extends in the inner cavity of the third anchoring part 310 may be U-shaped, V-shaped, or the like.

Please continue to refer to Fig. 9, the distal end of the connector 200 (i.e., the end of the first connection part 210 away from the second connection part 220) is provided with a limiting part 230 that has a diameter larger than the inner diameter of the inner cavity of the third anchoring part 310 and is located outside the inner cavity of the third anchoring part 310, so as to prevent the first connection part 210 from moving out of the inner cavity of the third anchoring part 310. Since the limiting part 230 is configured to prevent the first connection part 210 from moving out of the inner cavity of the third anchoring part 310, the connector 200 can be prevented from moving axially along the inner cavity of the third anchoring part 310. Therefore, the stability of the connection between the distal anchor 300 and the connector 200 is improved and the reliability of the anchoring device is improved. The assembling of the connector 200 with the distal anchor 300 and the formation of the limiting part 230 will be described in detail later.

Please continue to refer to Fig. 8, the anchoring body further includes a fourth anchoring part 320 that has a curved sheet-like structure and is configured for connecting the third anchoring part 310 and the bent structure. At the connection between the fourth anchoring part 320 and the third anchoring part 310, the fourth anchoring part 320 covers at least 1/2 of the circumferential surface of the anchoring body, so that the strength of the connection between the third anchoring part 310 and the fourth anchoring part 320 is ensured to avoid breakage during use. In addition, along the direction away from the third anchoring part 310, the portion of the circumferential surface of the anchoring body covered by the fourth anchoring part 320 decreases, so as to reduce stress concentration and stress strain.

Please continue to refer to Fig. 8. In this embodiment, the bent structure is implemented as a curved sheet-like structure and includes a bent part 330. The fourth anchoring part 320 is close to the bent part 330. An indent 321 is formed on the side of the fourth anchoring part 320, and can be formed by an inclined surface, but those skilled in the art may know that the indent can also be formed by an arc surface, a stepped surface, or other curved or straight surfaces. At the connection between the fourth anchoring part 320 and the bent part 330, the fourth anchoring part 320 covers at least 1/12 of the circumferential surface of the anchoring body. Arranging the bent structure on the distal anchor 300 can facilitate the fixing of the distal anchor 300 inside the prostate tissue and reduce the risk of displacement of the distal anchor 300.

Preferably, the bent structure further includes a fifth anchoring part 340. In the axial direction of the bent structure, the fifth anchoring part 340 has a length of 0.5mm-1mm. The angle formed between the fifth anchoring part 340 and the axis of the anchoring body is greater than 90° and less than 180°, or equal to 90°. The portion of the circumferential surface of the bent structure covered by the fifth anchoring part 340 is larger than the portion of the circumferential surface of the bent structure covered by the bent part 330. In some embodiments, the fifth anchoring part 340 covers at least 1/2 of the circumferential surface, and the bent part 330 may cover only about 1/12 of the circumferential surface. In addition, in some embodiments, the bent structure further includes a second transition section (not annotated in the figures), and the second transition section is configured to connect the bent part 330 and the fifth anchoring part 340.

Preferably, the various components of the distal anchor 300 are connected smoothly so that no sharp corners appear on the distal anchor 300 and thus stress concentration is reduced.

In the anchoring device of this embodiment, the connector 200 and the distal anchor 300 are molded and assembled before being implanted into the patient's body, while the connector 200 and the proximal anchor 100 will be connected after being implanted into the patient's body. Fig. 10 is a schematic diagram of implanting the anchoring device onto the prostate P of a patient according to an embodiment of the present invention, in which the distal anchor 300 is positioned on the distal tissue of the prostate P, the proximal anchor 100 is positioned on the proximal tissue of the prostate P, and the anchoring body of the distal anchor 300 is substantially parallel to the proximal anchor 100, so the connector 200 is L-shaped (also shown in Fig. 9).

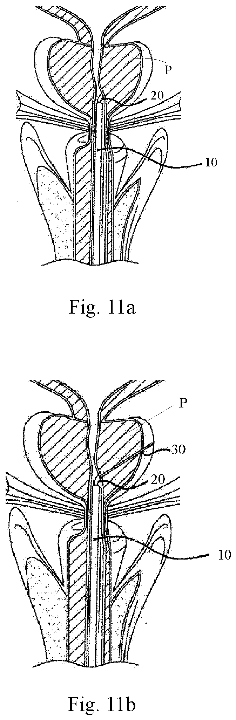

The specific process of using the anchoring device to treat benign prostatic hyperplasia will be described below with reference to Figs. 11a to 11g.

First, a sheath 10 moves forward and passes through the urethra such that the distal end of the sheath 10 is placed near the area where the urethra is obstructed by hypertrophic prostate tissue; and a distal anchor delivery device 20 is introduced through the sheath 10 so that the distal end of the distal anchor delivery device 20 emerges from the distal end of the sheath 10. Here, the distal anchor delivery device 20 may be placed in the sheath 10 after the sheath 10 is placed near the obstructed area in the urethra, or alternatively, the distal anchor delivery device may be preload in the sheath 10 before the sheath 10 is implanted into the urethra. Fig. 11a shows that the distal end of the distal anchor delivery device 20 emerges from the distal end of the sheath 10.

Next, a paracentesis needle 30 is introduced by the distal anchor delivery device 20, the paracentesis needle 30 passes through the distal anchor delivery device 20, and reaches a desired location outside the sheath 10 from the distal opening of the distal anchor delivery device 20. The paracentesis needle 30 further moves forward, and pierces the prostate tissue and emerges from the prostate capsule, as shown in Fig. 11b.

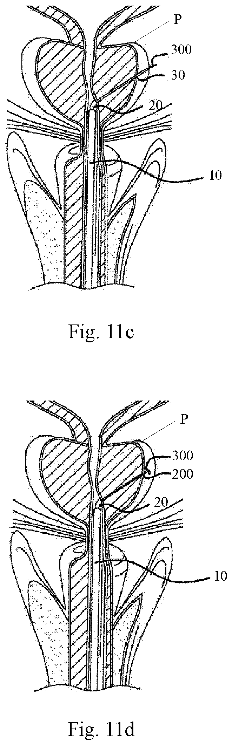

Next, the distal anchor 300 is passed through the distal end of the paracentesis needle 30, as shown in Fig. 11c.

Next, the paracentesis needle 30 is retrieved such that the paracentesis needle 30 is removed from the distal anchor delivery device 20 and the distal anchor 300 is released, as shown in Fig. 11d.

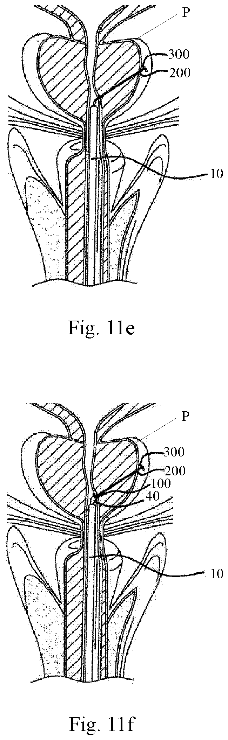

Next, the distal anchor delivery device 20 is removed from the sheath 10, and the position of the distal anchor 300 is adjusted, as shown in Fig. 11e.

Next, a proximal anchor delivery device 40 is introduced by the sheath 10, and the distal end of the proximal anchor delivery device 40 protrudes from the distal end of the sheath 10. Before the distal end of the proximal anchor delivery device 40 prodrudes from the distal end of the sheath 10, the connector 200 may pass through the proximal anchor 100 in the proximal anchor delivery device. At this time, the connector 200 and the proximal anchor 100 are still separated from each other, and the position of the connector 200 relative to the proximal anchor 100 can be adjusted in real time, as shown in Fig. 11f.

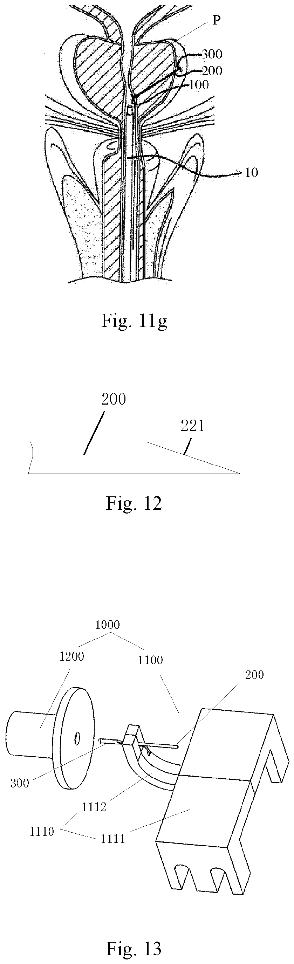

Next, the distal anchor 300 is pulled by means of the connector 200 to adjust the position of the distal anchor 300 relative to the proximal anchor 100, so as to shrink the hypertrophic prostate tissue. After that, the proximal anchor 100 is connected with the connector 200 so that the position of the proximal anchor 100 is relatively fixed, and the proximal anchor 100 is released from the proximal anchor delivery device 40. Thus, the proximal anchor 100 is positioned in the tissue, thereby implanting one anchoring device into the tissue, as shown in Fig. 11g.

After that, according to actual situations, the above operations are repeated to implant other anchoring devices into the prostate tissue. Generally, 4-6 anchoring devices need to be implanted, and these anchoring devices work together to treat benign prostatic hyperplasia.

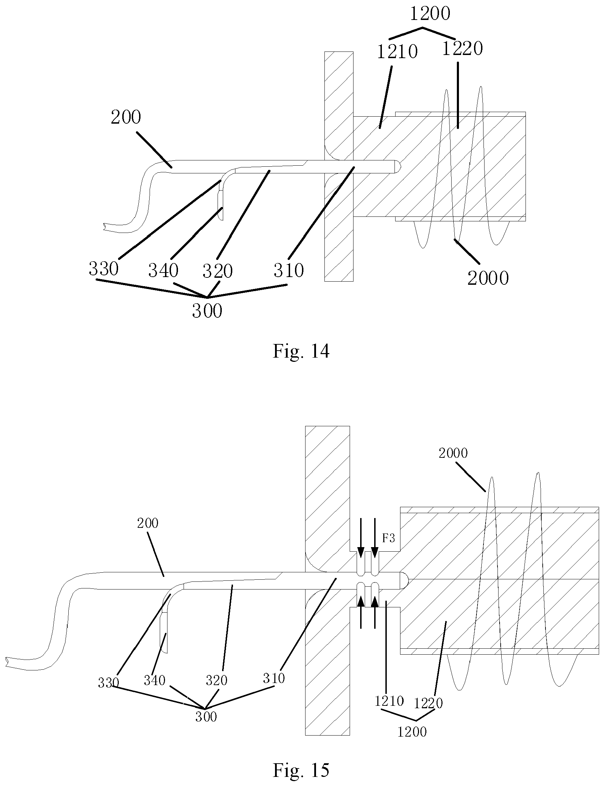

The present embodiment will describe the method for connecting the distal anchor 300 to the connector 200 with reference to Figs. 14-16.

Figs. 13 and 14 are schematic diagrams of an auxiliary tool 1000 for assembling the distal anchor 300 and the connector 200. As shown in Figs. 13 and 14, the auxiliary tool 1000 includes a fixture 1100 and a processing part 1200. The fixture 1100 includes two symmetrically arranged sub-fixtures 1110, and each of the sub-fixtures 1110 includes a base 1111 and a clamping arm 1112 extending from the base 1111. Recessed areas for clamping are formed on the opposite surfaces of the two clamping arms 1112. When the two sub-fixtures 1110 are connected with each other to form the fixture 1100, the two areas for clamping are aligned with each other for clamping the distal anchor 300. The processing part 1200 includes a crimping section 1210 and a heating section 1220 that are arranged along the axial direction of the processing part 1200. The crimping section 1210 is provided with a processing cavity extending along the axial direction thereof. The processing cavity has an opening, from which the third anchoring part 310 of the distal anchor 300 can be inserted into processing cavity. The surface of the bottom of the processing cavity (i.e., the end of the processing cavity opposite to the opening) is a part of a spherical surface, such as 1/2 of a spherical surface, and preferably the bottom of the processing cavity partially extends to the heating section 1220.

In this embodiment, the distal anchor 300 and the connector 200 are fixed with each other by crimping. The connector 200 has a rod-shaped structure, and the limiting part 230 is formed by hot melting. The assembling of the connector 200 and the distal anchor 300 includes the following steps.

First, the distal end of the connector 200 is inserted into the inner cavity of the third anchoring part 310 from one end thereof (i.e., the end connecting with the fourth anchoring part 320), and then protrudes out from the other end of the inner cavity. For ease of operation, the distal end of the connector 200 is processed to be a tip 221, as shown in Fig. 12. The length of the tip 221 is about 1 mm, and the length of the portion of the connector 200 prodruding out of the inner cavity is 3-4 mm.

Next, the distal anchor 300 through which the connector 200 is passed is fixed to the fixture 1100. The two sub-fixtures 1110 are respectively connected with pneumatic parts (not shown in the figures), and the pneumatic parts provide pressure to the sub-fixtures 1110, so that the two sub-fixtures 1110 clamp the distal anchor 300. Next, the third anchoring part 310 of the distal anchor 300 is inserted into the processing cavity of the processing part 1200, and the distal end of the connector 200 is located at the bottom of the processing cavity. It can be understood that the order of these two steps may be swapped in actual operation.

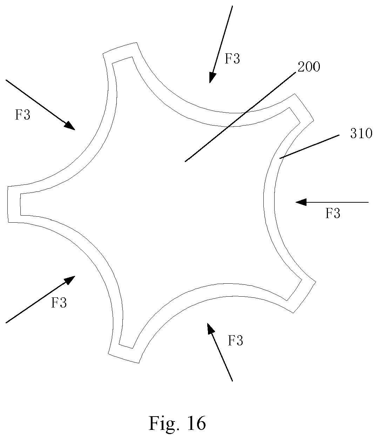

Next, the crimping process and the hot melting process are simultaneously performed. Specifically, a heating wire 2000 is provided on the outer surface of the heating section 1220 of the processing part 1200, and electrical energy is supplied to the heating wire 2000 to make the heating wire 2000 generate heat, and then the distal end of the connector 200 is melting. At the same time, a crimping device (not shown in the figures) is used to apply a force F3 to the crimping section 1210 in the circumferential direction thereof, so that the third anchoring part of the distal anchor 300 is fixed with the connector 200 by crimping. During this process, the forces that the distal anchor 300 and the connector 200 are subject to are shown in Figs. 15 and 16.

Next, the heating process is stopped, and the limiting part 230 is formed at the distal end of the connector 200 after the processing part 1200 is cooled.

In the above process, the crimping process and the hot-melting process are performed at the same time as an example for illustration. In practice, the crimping process and the hot-melting process may be performed one after another. That is to say, the crimping process may be performed before the hot-melting process is performed, or the hot-melting process may be performed before the crimping process is performed, which is not limited in the present invention.

In other embodiments, the connector 200 may alternatively have a rope-like structure. In this case, the limiting part 230 may be formed by knotting.

Although the present invention is disclosed above, it is not limited thereto. Various modifications and variations can be made in the present invention by those skilled in the art without departing from the spirit and scope of the invention. If modifications and variations of the present invention fall within the scope of the claims of the present invention and their equivalents, the present invention is also intended to include such modifications and variations.

## Claims

1. An anchor, comprising a first anchoring part and a second anchoring part extending from an end of the first anchoring part;
wherein the second anchoring part comprises a first anchoring sheet and a second anchoring sheet that are spaced apart from each other, and a clamping groove is formed between the first anchoring sheet and the second anchoring sheet; wherein the clamping groove comprises a buffer section, a clamping section and a first transition section that are arranged in sequence along a direction from the first anchoring part to the second anchoring part, the clamping section having a width larger than a width of the buffer section, the first transition section having a width gradually increasing along a direction away from the clamping section, a minimum width of the first transition section is smaller than the width of the clamping section; wherein the width refers to a distance between the anchoring sheet and the second anchoring sheet.

2. The anchor according to claim 1, wherein the clamping groove is configured to clamp a target object, and the buffer section has a length greater than a diameter of a radial section of the target object.

3. The anchor according to claim 2, wherein the minimum width of the first transition section is smaller than the diameter of the radial section of the target object, and a maximum width of the first transition section is larger than the diameter of the radial section of the target object.

4. The anchor according to claim 1, wherein, in an extension direction of the anchor, a ratio of a length of the first anchoring part to a length of the anchor is in a range of [1/10, 1/2].

5. The anchor according to any one of claims 1-4, wherein the clamping groove further comprises an open section that is arranged at an end of the first transition section away from the clamping section and has a width that is equal to a maximum width of the first transition section.

6. An anchoring device, comprising a connector, a distal anchor and the anchor according to any one of claims 1-5, the connector comprising a first connection part and a second connection part, the distal anchor fixedly connected with the first connection part, and the second anchoring part of the anchor configured to clamp the second connection part so that the anchor is connected to the second connection part.

7. The anchoring device of claim 6, wherein the distal anchor comprises an anchoring body and a bent structure extending from an end of the anchoring body, the anchoring body comprising a third anchoring part that is tubular;
wherein the first connection part is arranged in an inner cavity of the third anchoring part, and the first connection part and the third anchoring part are fixed to each other by crimping, so that a wall of the third anchoring part extends into the inner cavity to form a limiting recess, and a fixing groove matching with the limiting recess is formed on the first connection part.

8. The anchoring device according to claim 7, wherein a limiting part is provided at an end of the first connection part away from the second connection part, the limiting portion has a diameter larger than a diameter of the inner cavity, and the limiting part is located outside the inner cavity of the third anchoring part, so as to prevent the first connection part from moving out of the inner cavity of the third anchoring part.

9. The anchoring device according to claim 7, wherein the anchoring body further comprises a fourth anchoring part having a curved sheet-like structure and configured to connect the third anchoring part and the bent structure; at a connection between the fourth anchoring part the third anchoring part, the fourth anchoring part covers at least 1/2 of a circumferential surface of the anchoring body.

10. The anchoring device according to claim 9, wherein a portion of the circumferential surface that the fourth anchoring part covers gradually reduces in a direction away from the third anchoring part.

11. The anchoring device according to claim 10, wherein the bent structure comprises a bent part; at the connection between the bent part and the fourth anchoring part, the fourth anchoring part covers at least 1/12 of the circumferential surface of the anchoring body.

12. The anchoring device according to claim 11, wherein the bent structure further comprises a fifth anchoring part arranged at an end of the bent part away from the fourth anchoring part; an angle in a range of [90°, 180°) is formed between the fifth anchoring part and an axis of the anchoring body.

13. The anchoring device according to claim 12, wherein the bent structure is formed as a curved sheet-like structure, and the fifth anchoring part covers at least 1/2 of a circumferential surface of the bent structure.

14. The anchoring device according to claim 12, wherein in an axial direction of the bent structure, the fifth anchoring part has a length of 0.5mm-1mm.

15. The anchoring device according to any one of claims 9-14, wherein in an axial direction of the anchoring body, a ratio of a length of the third anchoring part to a length of the anchoring body is in a range of [1/10, 1/2].
